# DEMANDE DE BREVET EUROPEEN

(11) **EP 2 174 710 A1**
(43) Date de publication de la demande: **14.04.2010**
(21) Numéro de dépôt: 09290729.4
(22) Date de dépôt: 25.09.2009
(51) Int. Cl.: B01J 23/80, B01J 37/03, C10G 25/02, C07C 7/12, C07C 31/02, C10G 25/00

(54) **Mise en oeuvre de solides à base de ferrite de zinc dans un procédé de désulfuration profonde de charges oxygénées**

(30) Priorité: 10.10.2008 FR 0805624
(71) Demandeur: IFP, 92852 Rueil-Malmaison Cédex (FR)
(72) Inventeur: Baudot, Arnaud, 69390 Vernaison (FR); Huard, Thierry, 69360 Saint Symphorien d'Ozon (FR); Thomas, Michel, 69003 Lyon (FR)

(57) **Abrégé**

L'invention concerne un procédé de désulfuration d'une charge comprenant des composés oxygénés, des composés hydrocarbonés et des composés soufrés organiques, par captation de soufre sur une masse de captation comprenant des oxydes de fer ou des oxydes de zinc et plus de 20% poids de ferrite de zinc. Le procédé est opéré en présence d'hydrogène à une température comprise entre 200°C et 400°C.

## Description

Des charges comprenant des composés oxygénés peuvent être mis en contact de catalyseur pour la production d'hydrogène par des réactions de réformage à la vapeur, d'oxydation partielle, ou de réformage autotherme. Une réaction complémentaire de déplacement à la vapeur (réaction de "Water Gas Shift") peut accroître le rendement en hydrogène de manière significative. La présence d'impuretés, et notamment d'impuretés soufrées, dans la charge est cependant néfaste, car conduit à une désactivation progressive du ou des catalyseurs utilisés dans ces réactions.

Plusieurs procédés sont utilisés pour produire de l'hydrogène à partir de charges riches en alcools. On peut citer par exemple :
- La réaction d'oxydation partielle (POX : Partial Oxydation), effectuée avec de l'air en quantité contrôlée, exothermique, s'écrit dans le cas
   où l'éthanol est utilisé, selon l'équation chimique :

   o CH₃-CH₂-OH + ½O₂ => 2CO + 3H₂
- La réaction de réformage à la vapeur, endothermique, s'écrit quant à elle :

   ○ CH₃-CH₂-OH + H₂O => 2CO + 4H₂
- La réaction de réformage autotherme, qui est un couplage de ces deux réactions, peut-être considérée globalement comme adiabatique, l'exothermicité d'une réaction compensant plus ou moins l'endothermicité de l'autre.

Le gaz de synthèse obtenu est alors composé majoritairement de monoxyde de carbone et d'hydrogène. Il peut également contenir non seulement de l'azote qui provient de l'air, de la vapeur d'eau en excès, mais aussi des composés soufrés issus de la dégradation des composés soufrés initialement présents dans la charge.

Une réaction complémentaire de déplacement à la vapeur (W.G.S. :
"Water Gas Shift") peut permettre d'accroître sensiblement le rendement en hydrogène par oxydation du monoxyde de carbone du gaz de synthèse. Cette réaction s'écrit :

   CO + H₂O => CO₂ + H₂

Ces réactions chimiques s'effectuent en phase gazeuse, souvent à haute température, typiquement supérieure à 200 °C, souvent supérieures à 600°C, en présence de catalyseurs, souvent très sensibles à la présence de composés soufrés dans la charge qui peuvent alors conduire à leur désactivation progressive.

La purification ultime de l'hydrogène, par exemple après la réaction de déplacement à la vapeur, pour obtenir des puretés supérieures à 99,9 %, peut être effectuée par un procédé par adsorption de type P.S.A. (Pressure Swing Adsorption), ou par membrane métallique à base de palladium. Dans tous les cas, la présence d'impuretés soufrées, notamment de sulfure d'hydrogène, est néfaste dans ces procédés.

Pour des raisons légales, on ajoute aux charges riches en alcool des agents dénaturant, de manière à rendre ces charges impropres à la consommation alimentaire. La teneur des agents dénaturant est généralement comprise entre 1 et 10 % en masse de la teneur en alcool, et plus souvent entre 2 et 5 % en masse.

L'agent dénaturant peut être un condensat de gaz naturel ou une coupe hydrocarbure de type essence, gazole ou naphta. Cet agent dénaturant comprend généralement hormis les composés hydrocarbonés, des composés soufrés, de type mercaptans légers ou intermédiaires, ou des composés soufrés aromatiques comme le thiophène, le benzothiophène ou encore le dibenzothiophène et leur dérivés substitués. La teneur dans la charge en équivalent soufre est variable, mais elle est généralement inférieure à 2000 ppmS, et plus souvent inférieure à 500 ppmS, voire 100 ppmS, voire 50 ppmS.

La présence de ces composés soufrés, même à faible teneur, dans la charge peut s'avérer néfaste pour le bon fonctionnement du ou des catalyseurs suite à une désactivation progressive due à un empoisonnement. Par ailleurs, la purification poussée de l'hydrogène obtenu, soit par un procédé par adsorption de type P.S.A. (Pressure Swing adsorption), soit sur membrane à base de palladium, peut s'avérer également très problématique.

### Art antérieur:

De nombreux procédés de désulfuration sur solides, de charges liquides ou gazeuses, sont décrits dans l'art antérieur.

On peut citer par exemple des procédés de désulfuration de gaz naturel, avec retrait notamment de mercaptans légers, par utilisation de tamis moléculaire, généralement de type NaX (13X), sous pression, typiquement entre 50 et 100 bar, avec une étape régénération thermique à haute température, généralement de l'ordre de 300 °C. La demande de brevet WO 03/062177 (A1) décrit par exemple ce type de procédé.
Le brevet US2005/0109206 décrit notamment l'utilisation de tamis moléculaire pour retirer les mercaptans du gaz naturel avec une méthode de régénération incluant en particulier une étape de déplacement des mercaptans adsorbés à l'aide d'un gaz de purge enrichi en composés hydrocarbonés à plus de cinq atomes de carbone.
La demande de brevet US2006/0131216 décrit un procédé de désulfuration de charges hydrocarbures, comme un gaz naturel, mettant en jeu un premier solide réalisant l'hydrolyse, en présence de vapeur d'eau et d'un catalyseur comme l'alumine, le titane, ou la zircone, des composés soufrés de type COS et CS₂ en d'autres composés soufrés, notamment en H₂S, captés ensuite par un deuxième solide adsorbant, notamment de type oxyde de zinc ou de nickel.

On peut aussi citer également des procédés de désulfuration de charges liquides, par exemple de type essence ou gazole, par des procédés par adsorption ou chimisorption.

Le brevet US3620969 décrit par exemple l'utilisation d'un tamis moléculaire pour réaliser la désulfuration de coupes hydrocarbures liquides, avec une étape de régénération thermique à l'aide d'un gaz de purge contenant des traces d'humidité.

La demande de brevet US2007/261993A décrit un procédé permettant de désulfurer notamment les essences issues du craquage catalytique (FCC), impliquant une étape de distillation de l'essence en une fraction légère contenant au moins le thiophène, et une fraction lourde, la coupe légère étant désulfurée par un procédé par adsorption en phase liquide sur tamis moléculaire de type faujasite X, la coupe lourde étant désulfurée par un procédé d'hydrotraitement classique sur un catalyseur comprenant au moins un élément du groupe VIII (élément choisi parmi le groupe constitué du chrome, molybdène et tungstène), au moins en partie sous forme sulfure.

Le brevet US5882614 décrit l'utilisation des solides tels que l'oxyde de zinc et le nickel métallique pour désulfurer un gaz naturel contenant notamment des impuretés soufrées comme H₂S, COS et des mercaptans légers, préalablement à sa transformation en gaz de synthèse.

Le brevet US6159256 décrit une méthode de désulfuration d'une charge hydrocarbure par utilisation d'un catalyseur à base de nickel permettant d'obtenir un effluent purifié, le soufre ayant réagit avec le nickel pour former du sulfure de nickel.

Le brevet US6428685 décrit un procédé de désulfuration de coupes hydrocarbures, notamment de type essences ou gazoles, par utilisation d'un promoteur métallique à base de cuivre, cobalt, nickel, manganèse couplé avec un sel de calcium (sulfate, silicate, phosphate, aluminate).

La demande de brevet US2004/0091753 décrit un procédé de désulfuration d'une charge hydrocarbonée destinée à la production d'hydrogène, par adsorption des composés soufrés sur un premier solide opérant à température modérée, de type zéolithe, charbon, alumine activée, argile, silice-alumine, puis sur un solide à base de nickel opérant à haute température.

La demande de brevet US2003/0163013 décrit l'utilisation de solides microporeux, comme des zéolithes Y échangées avec des cations de métaux de transition (Cu, Ag), pour réaliser la désulfuration de coupes hydrocarbures liquides comme un essence, via le phénomène de π-complexation.

La demande de brevet US2003/0183803 revendique l'utilisation d'oxyde de cuivre et d'un promoteur métallique réduit pour réaliser la désulfuration de charges hydrocarbonées.

Enfin la demande de brevet US2002/0139718A1 revendique un procédé de désulfuration de charges hydrocarbonées liquides comme les essence ou les gazoles, par utilisation de nickel en présence d'un effluent oxygéné comme par exemple du méthanol, de l'éthanol, ou du MTBE.

### Description sommaire de l'invention

L'invention concerne un procédé de désulfuration d'une charge comprenant des composés oxygénés, des composés hydrocarbonés et des composés soufrés organiques, par captation de soufre sur une masse de captation comprenant des oxydes de fer ou des oxydes de zinc et plus de 20% poids de ferrite de zinc.

### Description détaillée de l'invention

L'invention concerne un procédé de désulfuration d'une charge comprenant des composés oxygénés, de préférence du méthanol et/ou de l'éthanol, des composés hydrocarbonés et des composés soufrés organiques, de préférence des composés soufrés aliphatiques, cycliques et/ou aromatiques, par captation de soufre sur une masse de captation comprenant des oxydes de fer ou des oxydes de zinc et plus de 20% poids de ferrite de zinc. Le procédé est opéré en présence d'hydrogène à une température comprise entre 200°C et 400°C.

La masse de captation comprend plus de 20% poids de ferrite de zinc, de manière plus préférée plus de 50% poids de ferrite de zinc, de manière plus préférée plus de 80% poids de ferrite de zinc, de manière plus préférée plus de 98% poids de ferrite de zinc, et de manière encore plus préférée plus de 99,5% poids de ferrite de zinc.
La pression est généralement comprise entre 0,2 et 3,5 MPa, de préférence entre 0,5 et 3 MPa, de manière encore préférée entre 0,5 et 1,5 MPa. La vitesse volumique horaire de charge à traiter est généralement comprise entre 0,1 h⁻¹ et 10 h⁻¹, de préférence entre 0,5 h⁻¹ et 5 h⁻¹. La vitesse volumique horaire ou VVH de charge liquide traitée est définie comme le débit volumique de charge liquide traitée sur le volume de composé solide. Le rapport volumique hydrogène/charge est généralement compris entre 5 et 500, de préférence entre 50 et 300. Les débits d'hydrogène et celui de charge liquide à traiter sont pris aux conditions normales.

Le procédé selon l'invention permet de réduire la teneur en soufre de la charge et de limiter les phénomènes de désactivation du ou des catalyseurs utilisés dans les réactions successives de production d'hydrogène.

Le soufre présent dans ces charges provient essentiellement des dénaturants de type condensat de gaz naturel, essence, naphta, gazole ou toute coupe hydrocarbure comprenant des composés soufrés. La teneur en agent dénaturant dans la charge comprenant des composés oxygénés est généralement comprise entre 1 et 10% massique, plus souvent entre 1 et 5 ou 1 et 2 % massique. La teneur en soufre de cet agent dénaturant est comprise entre 1 et 5000 ppm en équivalent soufre, et plus généralement entre 10 et 500 ppmS, voire même entre 10 et 100 ppmS.
Selon la nature du dénaturant, la nature des composés soufrés est variable. Dans le cas d'un condensat de gaz naturel, il s'agira essentiellement des mercaptans légers, comme les méthyl-, éthyl-, propyl- et butylmercaptan, ainsi que leurs isomères et dérivés substitués, ou bien un composé comme le tétrahydrothiophène (THT) qui peut aussi être ajouté comme agent odorant. Dans le cas d'une essence, les composés soufrés pourront être des mercaptans, linéaires, ramifiés ou cycliques, comportant par exemple de 4 à 10 atomes de carbone, ainsi que des composés aromatiques soufrés tels le thiophène et ses dérivés mono ou diméthylés, ou bien le benzothiophène et ses dérivés mono ou diméthylés. Dans le cas d'un gazole, les composés soufrés seront pour l'essentiel des composés aromatiques, comme le benzothiophène et ses dérivés mono ou diméthylés, et le dibenzothiophène et ses dérivés mono ou diméthylés, et notamment le 4,6-diméthyldibenzothiophène.

L'opération de désulfuration est réalisée de préférence en phase gazeuse. Selon une variante, elle peut être réalisée en phase liquide. Ceci est le cas lorsque la température de réaction est inférieure à la température critique du mélange alcool et hydrogène.

La masse de captation selon l'invention capte les molécules organiques soufrées, notamment avec les molécules considérées comme réfractaires aux opérations d'hydrodésulfuration classiques, telles que les benzo ou dibenzothiophènes alkylés.

L'oxyde mixte de type ferrite de zinc est généralement obtenu par une coprécipitation suivie d'une calcination. Le procédé de préparation ne nécessite pas d'étape intermédiaire d'imprégnation d'une seconde phase agissant comme promoteur. L'oxyde mixte ferrite de zinc est actif même avec des surfaces spécifiques inférieures à 10 m²/g. La synthèse selon l'invention d'une masse active à base de ferrite de zinc ne nécessite pas de protocole sophistiqué visant à développer une surface spécifique importante nécessaire à une réactivité élevée de ce solide vis-à-vis des molécules soufrées. Le procédé de préparation ne nécessite pas d'étape de réduction afin de rendre l'oxyde actif ni d'étape de dispersion du promoteur (par exemple un oxyde de fer ou de cuivre) sur l'oxyde. L'étape de réduction, par exemple à l'hydrogène, est généralement délicate à opérer industriellement en lit fixe du fait de l'exothermicité de la réaction.
Néanmoins, une étape de réduction préalable sous hydrogène ne sort pas du cadre de l'invention. Les conditions typiques de cette étape seraient, par exemple, une température comprise entre 300°C et 400°C, une pression comprise, par exemple, entre l'atmosphère et 10 bar.

Le procédé de préparation d'un oxyde mixte de type ferrite de zinc comprend généralement :
- une étape de coprécipitation d'un mélange de sels précurseurs de zinc II et de fer III, en présence d'une base, à un pH compris entre 6,1 et 6,9 et à une température comprise entre 30°C et 50°C
- une étape de filtration du précipité obtenu
- une étape de séchage pendant une durée comprise entre 12 et 24 heures, à une température comprise entre 125°C et 175°C
- une étape de calcination en présence d'oxygène à une température comprise entre 600°C et 700°C, pendant une durée comprise entre 1 heure et 3 heures.

Le solide proposé dans cette invention ne nécessite pas une étape d'activation préalable par l'hydrogène pour être actif.

Selon une variante, la ferrite de zinc peut être déposée sur un support, comme par exemple de l'alumine.
La teneur en alumine est alors préférentiellement inférieure à 80% en masse.

La ferrite de zinc de formule ZnFe₂O₄ présente généralement une structure cristalline de type franklinite.
La taille des cristallites de ferrite de zinc est généralement comprise entre 20 et 5000 Å, de préférence entre 100 et 1000 Å.

La surface spécifique de la ferrite de zinc, mesurée par la technique d'adsorption d'azote à 77K selon la méthode B.E.T., est généralement comprise entre 2 et 10 m²/g. La ferrite de zinc est active pour des surfaces spécifiques inférieures à 10 m²/g. La ferrite de zinc pourra être mise en oeuvre sous forme de poudre, de billes ou d'extrudés. La ferrite de zinc est préférentiellement opérée en lit fixe mais il est également possible de l'opérer en lit circulant.

Le volume micro et mésoporeux de la ferrite de zinc, déterminés par la même technique d'adsorption d'azote selon par exemple la méthode B.J.H. ou ses variantes, est inférieur à 0,15 cm³/g.
Le volume macroporeux de la ferrite de zinc, mesurée par la technique par intrusion et extrusion de mercure, également bien connue de l'homme de l'art, est inférieur à 0,025 cm³/g.
Ces techniques de caractérisation sont bien décrites par exemple dans l'ouvrage de S. Lowell et al., "Characterizatin of Porous Solids and Powders : Surface Area, Pore size And Density", Kluver Academic publishers, 2004.

### Exemples

### Exemple n°1 : préparation d'un solide adsorbant de type ferrite de zinc.

L'adsorbant est préparé par précipitation d'un mélange de solutions aqueuses de nitrate de zinc (II), nitrate de fer (III) et d'une solution aqueuse d'ammoniaque comme agent basifiant. Dans la solution nitratée, les concentrations massiques de Zinc et de Fer sont respectivement de 13 g/l et 22,5 g/l. La concentration massique en agent précipitant est de 225 g/l.

Au démarrage de la synthèse, un pied d'eau est introduit dans un réacteur en verre borosilicate double enveloppe puis chauffé à 40 °C, sous une puissance d'agitation d'environ 150 W/m³ libérée par un mobile à débit axial de type hélice à pales. Les précurseurs et la base sont ensuite introduits dans le réacteur via un système de pompage permettant de réguler les débits d'introduction et la durée de la synthèse. La maîtrise du pH est assurée par le débit de la pompe basique : il est maintenu constant à 6,5 ± 0,2 tout au long de la coprécipitation.

Lors de la réaction, une puissance d'agitation d'environ 75 W/m³ est appliquée dans le milieu réactionnel et une température de 40°C ± 2 °C est maintenu dans le réacteur via un bain thermostaté.

A la fin de la synthèse, le précipité est filtré à chaud sur Büchner. Le gâteau humide obtenu après 45 minutes de filtration est séché à l'étuve pendant 18h à une température de 150 °C. Le solide obtenu est ensuite calciné en présence d'oxygène moléculaire à une température de 650 °C durant 2h.

Le solide obtenu a été caractérisé par diffraction des rayons X via un diffractomètre à poudres de type Bragg-Brentano en configuration θ-θ. Les conditions d'enregistrement sont les suivantes : une tension de l'anticathode réglée à 35 kV, l'intensité dans le filament de l'anticathode fixée sur 35 mA, le pas d'échantillonnage égale à 0,05°2θ, le temps de comptage par pas fixé à 5s et un domaine angulaire allant de 2 à 72°2θ. Sur le diffractogramme expérimental obtenu sur notre solide, la position des raies est similaire à celle d'une structure cristallographique connue et répertoriée dans la base de donnée "Powder Diffraction File" correspondant à la Franklinite ZnFe204 (PDF N° 00-022-1012). Pour exemple, les positions des raies expérimentales les plus intenses sont les suivantes pour notre solide : 29,93°2θ - 35,27°2θ - 56,61°2θ - 62,15°2θ. Pour la Franklinite, elles sont à 29,92°2θ - 35,26°2θ - 56,63°2θ - 62,21°2θ. Quant au paramètre de maille (a = b = c dans le cas d'un système cubique), il est identique, i.e égale à 8,44 Å. Pour notre solide, la taille moyenne des cristallites de ferrite de zinc est de 410 ± 40 Å.

Une analyse semi-quantitative par fluorescence X a également été effectuée sur notre solide synthétisé. Les teneurs obtenues après correction d'une perte au feu effectuée à 550°C, 4h (PAF = 0,3 %) conduit aux teneurs suivantes : Wt% Fe = 42,48 ± 0,74 % et Wt% Zn = 23,18 ± 0,78 %.

Enfin, la surface spécifique développée par le solide a été estimée par une volumétrie à l'azote à basse température suivant les normes ASTM D 3663-84 ou NFX 11-621 : elle est égale à 6 ± 1 m²/g.

### Exemple n°2 : désulfuration par chimisorption sur ferrite de zinc d'une charge modèle éthanol 95% dénaturé par 5%, d'hexane et 60 ppm"S" de butanethiol.

Le deuxième exemple décrit l'utilisation du solide dans un réacteur de type lit fixe. 12 gramme de solide sous forme de poudre fabriquée selon la description de l'exemple n°1 sont introduits dans une colonne de 1 cm de diamètre intérieur et de volume utile 9 cm³. Selon la masse volumique du solide, de la laine de quartz et un inerte de granulométrie équivalente au solide testé sont ajoutés de façon symétrique dans le réacteur. La colonne est placée dans une étuve régulée en température. Avant le test, le solide est préalablement réduit sous flux d'hydrogène (5,7nL/hr) à haute température 380°C et sous une pression de 7 bar pendant 12 heures. Après refroidissement, la température de l'étuve est amenée à 250°C.

La régulation de la température est une régulation externe, avec mesures de la température de paroi de la colonne, ce qui permet de travailler sans doigt de gant et élimine tout chemin préférentiel dans la colonne.

L'effluent de sortie est maintenu en température et prélevé au moyen d'une boucle d'échantillonnage pour un suivi analytique en ligne. Les composés sont analysés par chromatographie en phase gazeuse équipée d'une détection FID et d'un analyseur PFPD.

Une charge modèle liquide constituée d'ethanol (95%poids), d'hexane (5% poids) et contenant 66 ppmS massiques de butanethiol est alimentée avec une WH de 4h⁻¹ grâce à une pompe seringue Gilson, puis vaporisé en présence d'hydrogène grâce à une dispositif dédié puis injecté dans le réacteur. La pression dans le réacteur est de 9 bar et le rapport hydrogène/charge en entrée du réacteur de 420.

Dès la mise en contact de la charge avec l'adsorbant, on observe une chute quasi immédiate de la teneur en soufre dans l'effluent du réacteur jusqu'à des valeurs inférieures à 5 ppm massique. Le profil de soufre en fonction du temps en sortie du réacteur est ainsi maintenu constant et inférieur à 1 mg/l avant le phénomène de perçage qui correspond à une remontée de la concentration en soufre jusqu'à atteindre la valeur de concentration en soufre en entrée lorsque l'adsorbant est complètement saturé en soufre. Avant perçage, aucune trace de soufre n'a pu être détectée par le détecteur PFPD du chromatographe en phase gazeuse.
On peut distinguer deux paramètres représentant les performances du solide à base de ferrite de zinc dans l'exemple décrit :
- la capacité dynamique, qui correspond à la teneur en soufre piégée sur l'adsorbant juste avant le perçage. Dans les conditions opératoires utilisées, la capacité dynamique en soufre de l'adsorbant à base de ferrite de zinc est de 9 % massique.
- la capacité à saturation, qui correspond à la capacité maximale en soufre de l'adsorbant mesurée après saturation. Dans les conditions opératoires utilisées, la capacité en soufre à saturation de l'adsorbant à base de ferrite de zinc est de 14 % massique.

## Revendications

1. Procédé de désulfuration d'une charge comprenant des composés oxygénés, des composés hydrocarbonés et des composés soufrés organiques, par captation de soufre sur une masse de captation comprenant des oxydes de fer ou des oxydes de zinc et plus de 20% poids de ferrite de zinc, ledit procédé étant opéré en présence d'hydrogène à une température comprise entre 200°C et 400°C.

2. Procédé selon la revendication 1 dans lequel la pression est comprise entre 0,2 et 3,5 MPa.

3. Procédé selon l'une des revendications 1 ou 2 dans lequel la pression est comprise entre 0,5 et 3 MPa.

4. Procédé selon l'une des revendications 1 à 3 dans lequel la vitesse volumique horaire de charge à traiter est comprise entre 0,1 h⁻¹ et 10 h⁻¹.

5. Procédé selon l'une des revendications 1 à 4 dans lequel le rapport volumique hydrogène/charge est compris entre 5 et 500.

6. Procédé selon l'une des revendications 1 à 5 dans lequel la charge comprend des composés soufrés aliphatiques cycliques et/ou aromatiques.

7. Procédé selon l'une des revendications 1 à 6 dans lequel le composé comprend plus de 80% poids de ferrite de zinc.

8. Procédé selon l'une des revendications 1 à 7 dans lequel le composé comprend plus de 98% poids de ferrite de zinc.

9. Procédé selon l'une des revendications 1 à 8 dans lequel les composés oxygénés sont du méthanol et/ou de l'éthanol.
